# EUROPEAN PATENT SPECIFICATION

(11) **EP 3 212 599 B1**
(45) Date of publication and mention of the grant of the patent: **30.12.2020**
(21) Application number: 15854561.6
(22) Date of filing: 27.10.2015
(51) Int. Cl.: C07C 1/22, C07C 1/20, C07C 5/327, C07C 2/66, C07C 9/14, C07C 11/02, C07C 15/107, C07C 67/08, C07C 69/52, C07C 303/26, C07C 309/67, C11C 1/04, C11C 3/00

(54) **METHODS FOR CO-PRODUCTION OF ALKYLBENZENE AND AN OLEOCHEMICAL FROM NATURAL OILS**
VERFAHREN ZUR GLEICHZEITIGEN HERSTELLUNG VON ALKYLBENZOL UND EINEM OLEOCHEMISCHEN PRODUKT AUS NATURÖLEN
PROCÉDÉS DE CO-PRODUCTION D'ALKYLBENZÈNE ET DE PRODUIT OLÉOCHIMIQUE À PARTIR D'HUILES NATURELLES

(30) Priority: 29.10.2014 US 201414527592
(43) Date of publication of application: 06.09.2017
(73) Proprietor: UOP LLC, Des Plaines, Illinois 60017-5017 (US)
(72) Inventor: ELLIG, Daniel L., Des Plaines, Illinois 60017-5017 (US); BOZZANO, Andrea G., Des Plaines, Illinois 60017-5017 (US)
(74) Representative: Boult Wade Tennant LLP
(86) International application number: PCT/US2015/057463
(87) International publication number: WO 2016/069527

(56) References cited:
- WO-A2-2014/111598
- US-A1- 2013 079 570
- US-A1- 2013 079 573
- US-A1- 2013 317 268
- US-A1- 2013 317 268
- OGOSHI T. ET AL.: '«Soap and Related Products: Palm and Lauric Oil»' JAOCS vol. 62, no. 2, 1985, pages 331 - 332, XP009502660

## Description

### TECHNICAL FIELD

The technical field generally relates to methods for co-production of alkylbenzene and oleochemicals, and more particularly relates to methods for producing renewable alkylbenzene and an oleochemical from natural oils.

### BACKGROUND

Linear alkylbenzenes are organic compounds with the formula C₆H₅CₙH₂ₙ₊₁. While n can have any practical value, current commercial use of alkylbenzenes requires that n lie between 10 and 16, or more specifically between 10 and 13, between 12 and 15, or between 12 and 13. These specific ranges are often required when the alkylbenzenes are used as intermediates in the production of surfactants for detergents. Because the surfactants created from alkylbenzenes are biodegradable, the production of alkylbenzenes has grown rapidly since their initial uses in detergent production in the 1960s.

While detergents made utilizing alkylbenzene-based surfactants are biodegradable, processes for creating alkylbenzenes are not based on renewable sources. Specifically, alkylbenzenes are typically produced from kerosene extracted from the earth. Due to the growing environmental concerns over fossil fuel extraction and economic concerns over exhausting fossil fuel deposits, there is support for using an alternate source for biodegradable surfactants in detergents and in other industries.

There is also an increasing demand for the use of bio-sourced and biodegradable products in other segments of the chemical industry. For example, demand is rising for oleochemicals, which are chemical compounds derived from oils or fats from animal, plant or fungus sources. Oleochemicals may be used in the form of fatty alcohols, fatty acids, glycerin, amines, and methyl esters. Regardless of form, oleochemicals typically exhibit low toxicity and are suitable for applications where toxicity is of importance. Use in surfactants, soaps, detergents, lubricants and other downstream renewable chemicals may further increase demand for oleochemicals.

Accordingly, it is desirable to identify new sources of linear alkylbenzenes and oleochemicals. Further, it is desirable to provide methods and systems that provide renewable alkylbenzenes and oleochemicals. Furthermore, other desirable features and characteristics will become apparent from the subsequent detailed description and the appended claims, when taken in conjunction with the accompanying drawing and this background.

US 2013/317268 relates to methods for co-production of alkylbenzene and biofuel from natural oils.

WO 2014/111598 relates to a process for the production of bio-naphtha from complex mixtures of natural occurring fats and oils.

### SUMMARY

The invention is in accordance with the appended claims.

In another exemplary embodiment disclosed herein, a method is provided for co-production of an alkylbenzene product and an oleochemical product from natural oil source triglycerides. The method includes fat splitting the natural oil source triglycerides to form a stream comprising glycerol and fatty acids. The method includes fractionating the stream to separate a first portion of fatty acids and a second portion of fatty acids. The method deoxygenates the first portion of fatty acids to form normal paraffins, dehydrogenates the normal paraffins to provide mono-olefins, alkylates benzene with the mono-olefins under alkylation conditions to provide an alkylation effluent comprising alkylbenzenes and benzene, and isolates the alkylbenzenes to provide the alkylbenzene product. The method includes processing the second portion of fatty acids to form the oleochemical product.

In accordance with another embodiment disclosed herein, a method for co-production of an alkylbenzene product and an oleochemical product from a natural oil includes deoxygenating a first portion of fatty acids with hydrogen to form a stream comprising paraffins. The method includes dehydrogenating the paraffins to provide mono-olefins and hydrogen, recycling the hydrogen to support deoxygenating the first portion of fatty acids; alkylating benzene with the mono-olefins under alkylation conditions to provide an alkylation effluent comprising alkylbenzenes and benzene; and isolating the alkylbenzenes to provide the alkylbenzene product. The method further includes processing a second portion of fatty acids to form the oleochemical product.

### BRIEF DESCRIPTION OF THE DRAWING

Embodiments of methods for co-production of alkylbenzene and oleochemical products from natural oils will hereinafter be described in conjunction with the following drawing figure wherein:

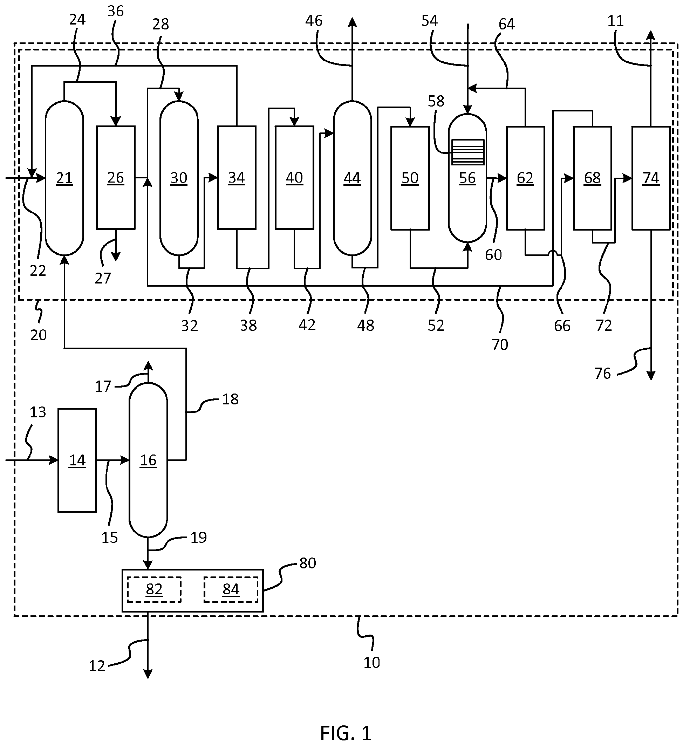

FIG. 1 schematically illustrates an apparatus for co-production of alkylbenzene and an oleochemical in accordance with an exemplary embodiment.

### DETAILED DESCRIPTION

The following Detailed Description is merely exemplary in nature and is not intended to limit the methods for co-production of an alkylbenzene and an oleochemical from natural oils. Furthermore, there is no intention to be bound by any theory presented in the preceding Background or the following Detailed Description.

Various embodiments contemplated herein relate to methods and systems for co-production of an alkylbenzene and an oleochemical from natural oils. In FIG. 1, an exemplary apparatus 10 for producing an alkylbenzene 11 and an oleochemical 12 from a natural oil feed 13 is illustrated. As used herein, natural oils are those derived from animal, plant or fungal matter, and are often referred to as renewable oils. Natural oils are not based on kerosene or other fossil fuels. In certain embodiments, the natural oils include one or more of palm kernel oil, coconut oil, babassu oil, castor oil, cooking oil, and other vegetable, nut or seed oils. The natural oils typically comprise triglycerides, free fatty acids, or a combination of triglycerides and free fatty acids.

In the illustrated embodiment, the natural oil feed 13 is delivered to a fat splitting unit 14. In the fat splitting unit 14, the triglycerides are split into free fatty chains. Specifically, fat splitting occurs according to the equation: one mole triglyceride + 3 moles water = one mole glycerol + 3 moles of fatty acid. A stream of fatty chains and glycerol 15 is formed by the fat splitting unit 14 and is fed to a separator 16. The separator 16 may be a multi-stage fractionation unit, distillation system or similar known apparatus. In any event, the separator 16 separates a stream of glycerol 17, a first portion 18 of fatty chains and a second portion 19 of fatty chains. Exemplary embodiments may include a separator for removing glycerol from stream 15 before entering separator 16. In certain embodiments, the first portion of fatty chains 18 has carbon chain lengths of C10 to C14. In other embodiments, the first portion of fatty chains 18 has carbon chain lengths having a lower limit of CL, where L is an integer from four (4) to thirty-one (31), and an upper limit of CU, where U is an integer from five (5) to thirty-two (32). The second portion of fatty chains 19 may have carbon chains shorter than, longer than, or a combination of shorter and longer than, the chains of the first portion of fatty chains 18. In an exemplary embodiment, the first portion of fatty chains 18 comprises C10 to C13 fatty chains and the second portion of fatty chains 19 comprises fatty chains with C9- fatty chains, i.e., C9 and shorter chains, and C14+ fatty chains, i.e., C14 and longer chains. While shown as a single stream exiting the separator 16, in such an embodiment, the second portion of fatty chains 19 includes an upper or light draw of C9- chains and a lower or heavier draw of C14+ chains from the separator 16, while the first portion of fatty chains 18 would be taken as a side draw between the upper and lower draws.

An exemplary first portion of fatty chains 18 includes no more than 2 weight percent (wt%) C9- fatty chains and no more than 1 wt% C14+ fatty chains. Further, an exemplary first portion of fatty chains 18 includes at least 97 wt% of C10 to C13 chains. C10 to C13 chains are particularly suited for the production of alkylbenzene, and the separation of C10 to C13 chains provides for efficient processing to form alkylbenzene and for the efficient processing of the remaining chains to form oleochemicals.

As shown in FIG. 1, the first portion of fatty chains 18 is introduced to an alkylbenzene production unit 20. Specifically, the first portion of fatty chains 18 is fed to a deoxygenation unit 21 which also receives a hydrogen feed 22. In the deoxygenation unit 21, the first portion of fatty chains 18 is deoxygenated and the fatty chains are converted into normal paraffins.

In FIG. 1, a deoxygenated stream 24 containing normal paraffins, water, carbon monoxide, carbon dioxide and propane exits the deoxygenation unit 21 and is fed to a separator 26. The separator 26 may be a multi-stage fractionation unit, distillation system or similar known apparatus. The separator 26 removes the water, carbon monoxide, carbon dioxide, and propane as stream 27 from the deoxygenated stream 24. While a single stream 27 is illustrated for simplicity, the water, carbon monoxide, carbon dioxide, and propane may be removed in separate streams. As shown, removal of the water, carbon monoxide, carbon dioxide, and propane by the separator 26 forms a normal paraffin stream 28. The normal paraffin stream 28 is fed to a dehydrogenation unit 30 in the alkylbenzene production unit 20. In the dehydrogenation unit 30, the normal paraffins are dehydrogenated into mono-olefins of the same carbon numbers as the paraffins. Typically, dehydrogenation occurs through known catalytic processes, such as the conventional Pacol process. Di-olefins (i.e., dienes) and aromatics are also produced as an undesired result of the dehydrogenation reactions.

In FIG. 1, a dehydrogenated stream 32 exits the dehydrogenation unit 30, and the dehydrogenated stream 32 comprises mono-olefins and hydrogen as well as some di-olefins and aromatics. The dehydrogenated stream 32 is delivered to a phase separator 34 for removing the hydrogen from the dehydrogenated stream 32. As shown, the hydrogen exits the phase separator 34 in a recycle stream of hydrogen 36 that can be added to the hydrogen feed 18 to support the deoxygenation process upstream.

At the phase separator 34, a liquid stream 38 is formed and comprises the mono-olefins as well as di-olefins and aromatics formed during dehydrogenation. The liquid stream 38 exits the phase separator 34 and enters a selective hydrogenation unit 40, such as a DeFine reactor. The hydrogenation unit 40 selectively hydrogenates at least a portion of the di-olefins in the liquid stream 38 to form additional mono-olefins. As a result, an enhanced stream 42 is formed with an increased mono-olefin concentration as compared to the liquid stream 38.

As shown, the enhanced stream 42 passes from the hydrogenation unit 40 to a lights separator 44, such as a stripper column, which removes a light end stream 46 containing any lights, such as butane, propane, ethane and methane, that resulted from cracking or other reactions during upstream processing. With the lights removed, stream 48 is formed and may be delivered to an aromatic removal apparatus 50 that removes aromatics from the stream 48 and forms a stream rich in mono-olefins 52.
As referred to herein, "rich" means that the stream at issue includes at least 50 weight % of the referenced compounds.

In FIG. 1, the stream of mono-olefins 52 and a stream of benzene 54 are fed into an alkylation unit 56. The alkylation unit 56 holds a catalyst 58, such as a solid acid catalyst, that supports alkylation of the benzene 54 with the mono-olefins 52. Hydrogen fluoride (HF) and aluminum chloride (AlCl₃) are two major catalysts in commercial use for the alkylation of benzene with linear mono-olefins and may be used in the alkylation unit 56. As a result of alkylation, alkylbenzene, typically called linear alkylbenzene (LAB), is formed and is present in an alkylation effluent 60.

To optimize the alkylation process, surplus amounts of benzene 54 are supplied to the alkylation unit 56. Therefore, the alkylation effluent 60 exiting the alkylation unit 56 contains alkylbenzene and unreacted benzene. Further the alkylation effluent 60 may also include some unreacted paraffins. In FIG. 1, the alkylation effluent 60 is passed to a benzene separation unit 62, such as a fractionation column, for separating the unreacted benzene from the alkylation effluent 60. This unreacted benzene exits the benzene separation unit 62 in a benzene recycle stream 64 that is delivered back into the alkylation unit 56 to reduce the volume of fresh benzene needed in stream 54.

As shown, a benzene-stripped stream 66 exits the benzene separation unit 62 and enters a paraffinic separation unit 68, such as a fractionation column. In the paraffinic separation unit 68, unreacted paraffins are removed from the benzene-stripped stream 66 in a recycle paraffin stream 70, and are routed to and mixed with the normal paraffin stream 28 before dehydrogenation as described above.

Further, an alkylbenzene stream 72 is separated by the paraffinic separation unit 68 and is fed to an alkylate separation unit 74. The alkylate separation unit 74, which may be, for example, a multi-column fractionation system, separates a heavy alkylate bottoms stream 76 from the alkylbenzene stream 72.

As a result of the post-alkylation separation processes, the linear alkylbenzene product 12 is isolated and exits the apparatus 10. It is noted that such separation processes are not necessary in all embodiments in order to isolate the alkylbenzene product 12. For instance, the alkylbenzene product 12 may be desired to have a wide range of carbon chain lengths and not require any fractionation to eliminate carbon chains longer than desired, i.e., heavies, or carbon chains shorter than desired, i.e., lights. Further, the fractionation performed at separator 16 may be sufficient such that no further fractionation is necessary despite the desired chain length range.

In certain embodiments, the natural oil source is castor, and the feed 13 comprises castor oils. Castor oils consist essentially of C₁₈ fatty acids with an additional, internal hydroxyl groups at the carbon-12 position. During fat splitting of a feed 13 comprising castor oil, it has been found that some portion of the carbon chains are cleaved at the carbon-12 position. Thus, deoxygenation creates a group of lighter C₁₀ to C₁₁ chains resulting and a group of non-cleaved heavier C₁₇ to C₁₈ chains. The first portion of fatty chains 18 may be rich in the lighter chains and the second portion of fatty chains 19 may be rich in the heavier chains. It should be noted that while castor oil is shown as an example of an oil with an additional internal hydroxyl group, others may exist. Also, it may be desirable to engineer genetically modified organisms to produce such oils by design. As such, any oil with an internal hydroxyl group may be a desirable feed oil.

The second portion of fatty chains 19 is not optimal for forming linear alkylbenzene. Thus, the stream of second portion of fatty chains 19 formed by the separator 16 are utilized herein to produce a different commercially valuable and renewable stream. As a result, utilization of the feed 13 is maximized.

As shown in FIG. 1, the second portion of fatty chains 19 is fed to an oleochemical production apparatus 80 for producing the oleochemical product 12, such as esters, alcohols, alkoxylates, ether sulfates, ether phosphates, sulfosuccinates, and/or other oleochemicals. In an exemplary embodiment, the oleochemical production apparatus 80 includes units 82 and 84 for processing the second portion of fatty chains 19. While the oleochemical production apparatus 80 is illustrated as including two processing units 82 and 84, more or fewer processing units may be included in the oleochemical production apparatus 80. In an exemplary process, the second portion of fatty chains 19 is fed to an esterification unit 82. The esterification unit 82 forms fatty acid methyl esters that are then fed to a sulfonation unit 84. The sulfonation unit 84 forms α sulfo-fatty acid esters, such as methyl ester sulfonate, as the oleochemical product 12.

Typically, no further deoxygenation is needed in the oleochemical production apparatus 80. Rather, in the apparatus 80, the second portion of fatty chains 19 are processed as selected for the desired oleochemical product 12. For example, the second portion of fatty chains 19 may undergo esterification, sulfonation, amidation, ethoxylation, hydrogenation, sulfation, epoxidation, chlorination, conjugation, fractionation, distillation, hardening, and bleaching and/or other processing to form the desired oleochemical product 12.

### SPECIFIC EMBODIMENTS

While the following is described in conjunction with specific embodiments, it will be understood that this description is intended to illustrate and not limit the scope of the preceding description and the appended claims.

A first embodiment of the invention is a method for co-production of an alkylbenzene product and an oleochemical product from a natural oil, the method comprising fat splitting the natural oil to form a stream of free fatty acids; fractionating the stream of free fatty acids to separate a first portion of free fatty acids and a second portion of free fatty acids; processing the first portion of free fatty acids to provide the alkylbenzene product; and processing the second portion of free fatty acids to form the oleochemical product; wherein processing the first portion of free fatty acids to provide the alkylbenzene product comprises deoxygenating the first portion of free fatty acids to produce normal paraffins; dehydrogenating the normal paraffins to provide mono-olefins; alkylating benzene with the mono-olefins under alkylation conditions to provide an alkylation effluent comprising alkylbenzenes and benzene; and isolating the alkylbenzenes to provide the alkylbenzene product; and wherein processing the second portion of free fatty acids to form the oleochemical product comprises performing an esterification process and a sulfonation process to form a methyl ester sulfonate product. An embodiment of the invention is one, any or all of prior embodiments in this paragraph up through the first embodiment in this paragraph wherein fractionating the stream comprises separating C₁₀ to C₁₃ free fatty acids as the first portion of free fatty acids and C₁₄₊ free fatty acids as the second portion of free fatty acids. An embodiment of the invention is one, any or all of prior embodiments in this paragraph up through the first embodiment in this paragraph wherein fractionating the stream comprises separating C₁₀ to C₁₃ free fatty acids as the first portion of free fatty acids, separating C₉₋ free fatty acids as the second portion of free fatty acids, and separating C₁₄₊ free fatty acids as a third portion of free fatty acids. An embodiment of the invention is one, any or all of prior embodiments in this paragraph up through the first embodiment in this paragraph wherein fractionating the stream comprises separating C₁₀ to C₁₃ free fatty acids as the first portion of free fatty acids and wherein the first portion of free fatty acids comprises at least 97 wt% C₁₀ to C₁₃ free fatty acids. An embodiment of the invention is one, any or all of prior embodiments in this paragraph up through the first embodiment in this paragraph wherein fractionating the stream comprises separating C₁₀ to C₁₃ free fatty acids as the first portion of free fatty acids and wherein the first portion of free fatty acids comprises no more than 2 wt% C₉₋ free fatty acids. An embodiment of the invention is one, any or all of prior embodiments in this paragraph up through the first embodiment in this paragraph wherein fractionating the stream comprises separating C₁₀ to C₁₃ free fatty acids as the first portion of free fatty acids and wherein the first portion of free fatty acids comprises no more than 1 wt% C₁₄₊ free fatty acids. An embodiment of the invention is one, any or all of prior embodiments in this paragraph up through the first embodiment in this paragraph further comprising providing palm kernel oil or coconut oil as the natural oil. An embodiment of the invention is one, any or all of prior embodiments in this paragraph up through the first embodiment in this paragraph wherein the natural oil comprises fatty acids with internal hydroxyl groups, and wherein deoxygenating the natural oil causes cleaving and provides the first portion of free fatty acids and the second portion of free fatty acids.

Also disclosed herein is a method for co-production of an alkylbenzene product and an oleochemical product from natural oil source triglycerides comprising fat splitting the natural oil source triglycerides to form a stream comprising glycerol and fatty acids; fractionating the stream to separate a first portion of fatty acids and a second portion of fatty acids; deoxygenating the first portion of fatty acids to form normal paraffins; dehydrogenating the normal paraffins to provide mono-olefins; alkylating benzene with the mono-olefins under alkylation conditions to provide an alkylation effluent comprising alkylbenzenes and benzene; isolating the alkylbenzenes to provide the alkylbenzene product; and processing the second portion of fatty acids to form the oleochemical product. An embodiment of the disclosure is one, any or all of prior embodiments in this paragraph up through the second embodiment in this paragraph wherein fractionating the stream comprises separating C₁₀ to C₁₃ fatty acids as the first portion of fatty acids and C₁₄₊ fatty acids as the second portion of fatty acids. An embodiment of the disclosure is one, any or all of prior embodiments in this paragraph up through the second embodiment in this paragraph wherein fractionating the stream comprises separating C₁₀ to C₁₃ fatty acids as the first portion of fatty acids and C9- fatty acids and C₁₄₊ fatty acids as the second portion of fatty acids. An embodiment of the disclosure is one, any or all of prior embodiments in this paragraph up through the second embodiment in this paragraph wherein fractionating the stream comprises separating C₁₀ to C₁₃ fatty acids as the first portion of fatty acids and wherein the first portion of fatty acids comprises at least 97 wt% C₁₀ to C₁₃ fatty acids. An embodiment of the disclosure is one, any or all of prior embodiments in this paragraph up through the second embodiment in this paragraph wherein fractionating the stream comprises separating C₁₀ to C₁₃ fatty acids as the first portion of fatty acids and wherein the first portion of fatty acids comprises no more than 2 wt% C₉₋ fatty acids. An embodiment of the disclosure is one, any or all of prior embodiments in this paragraph up through the second embodiment in this paragraph wherein fractionating the stream comprises separating C₁₀ to C₁₃ fatty acids as the first portion of fatty acids and wherein the first portion of fatty acids comprises no more than 1 wt% C₁₄₊ fatty acids. An embodiment of the disclosure is one, any or all of prior embodiments in this paragraph up through the second embodiment in this paragraph wherein processing the second portion of fatty acids to form the oleochemical product comprises performing an esterification process and a sulfonation process to form a methyl ester sulfonate product.

Also disclosed herein is a method for co-production of an alkylbenzene product and an oleochemical product from a natural oil comprising fat splitting the oil to form fatty acids; deoxygenating a first portion of fatty acids with hydrogen to form a stream comprising paraffins; dehydrogenating the paraffins to provide mono-olefins and hydrogen; recycling the hydrogen to support deoxygenating the first portion of fatty acids; alkylating benzene with the mono-olefins under alkylation conditions to provide an alkylation effluent comprising alkylbenzenes and benzene; isolating the alkylbenzenes to provide the alkylbenzene product; and processing a second portion of fatty acids to form the oleochemical product. An embodiment of the disclosure is one, any or all of prior embodiments in this paragraph up through the third embodiment in this paragraph wherein the first portion of fatty acids comprises C₁₀ to C₁₃ fatty acids. An embodiment of the disclosure is one, any or all of prior embodiments in this paragraph up through the third embodiment in this paragraph wherein the first portion of fatty acids comprises at least 97 wt% C₁₀ to C₁₃ fatty acids, no more than 2 wt% C₉₋ fatty acids, and no more than 1 wt% C₁₄₊ fatty acids.

In the foregoing, all temperatures are set forth in degrees Celsius and, all parts and percentages are by weight, unless otherwise indicated.

## Claims

1. A method for co-production of an alkylbenzene product (11) and an oleochemical product (12) from a natural oil (13), the method comprising:
fat splitting the natural oil to form a stream of free fatty acids (15);
fractionating the stream of free fatty acids to separate a first portion (18) of free fatty acids and a second portion (19) of free fatty acids;
processing the first portion of free fatty acids to provide the alkylbenzene product; and
processing the second portion of free fatty acids to form the oleochemical product;
wherein processing the first portion of free fatty acids comprises:
deoxygenating the first portion of free fatty acids to produce normal paraffins (28);
dehydrogenating the normal paraffins to provide mono-olefins (52);
alkylating benzene (54) with the mono-olefins under alkylation conditions to provide an alkylation effluent (60) comprising alkylbenzenes and benzene; and
isolating the alkylbenzenes to provide the alkylbenzene product; and
wherein processing the second portion of free fatty acids comprises performing an esterification process and a sulfonation process to form a methyl ester sulfonate product.

2. The method of claim 1 wherein fractionating the stream comprises separating C₁₀ to C₁₃ free fatty acids as the first portion of free fatty acids and C₁₄₊ free fatty acids as the second portion of free fatty acids.

3. The method of claim 1 wherein fractionating the stream comprises separating C₁₀ to C₁₃ free fatty acids as the first portion of free fatty acids, and separating C₉₋ free fatty acids and C₁₄₊ free fatty acids as the second portion of free fatty acids.

4. The method of claim 1 wherein fractionating the stream comprises separating C₁₀ to C₁₃ free fatty acids as the first portion of free fatty acids and wherein the first portion of free fatty acids comprises at least 97 wt% C₁₀ to C₁₃ free fatty acids.

5. The method of claim 4 wherein fractionating the stream comprises separating C₁₀ to C₁₃ free fatty acids as the first portion of free fatty acids and wherein the first portion of free fatty acids comprises no more than 2 wt% C₉₋ free fatty acids.

6. The method of claim 5 wherein fractionating the stream comprises separating C₁₀ to C₁₃ free fatty acids as the first portion of free fatty acids and wherein the first portion of free fatty acids comprises no more than 1 wt% C₁₄₊ free fatty acids.

7. The method of claim 1 further comprising providing palm kernel oil or coconut oil as the natural oil.

8. The method of claim 1 wherein the natural oil comprises fatty acids with internal hydroxyl groups, and wherein deoxygenating the natural oil causes cleaving and provides the first portion of free fatty acids and the second portion of free fatty acids.

## Patentansprüche

1. Verfahren zur gleichzeitigen Herstellung eines Alkylbenzolprodukts (11) und eines oleochemischen Produkts (12) aus einem natürlichen ÖI (13), wobei das Verfahren umfasst:
Fett, das das natürliche ÖI aufspaltet, um einen Strom von freien Fettsäuren (15) zu bilden;
Fraktionieren des Stroms von freien Fettsäuren, um einen ersten Teil (18) von freien Fettsäuren und einen zweiten Teil (19) von freien Fettsäuren zu trennen;
Verarbeiten des ersten Teils von freien Fettsäuren, um das Alkylbenzolprodukt bereitzustellen; und
Verarbeiten des zweiten Teils von freien Fettsäuren, um das oleochemische Produkt zu bilden;
wobei die Verarbeitung des ersten Teils von freien Fettsäuren umfasst:
Desoxidation des ersten Teils von freien Fettsäuren, um normale Paraffine (28) herzustellen;
Dehydrierung der normalen Paraffine, um Monoolefine (52) bereitzustellen;
Alkylieren von Benzol (54) mit den Monoolefinen unter Alkylierungsbedingungen, um ein Alkylierungsabwasser (60) bereitzustellen, das Alkylbenzole und Benzol umfasst; und
Isolieren der Alkylbenzole, um das Alkylbenzolprodukt bereitzustellen; und
wobei das Verarbeiten des zweiten Teils von freien Fettsäuren das Durchführen eines Veresterungsverfahrens und eines Sulfonierungsverfahrens umfasst, um ein Methylestersulfonatprodukt zu bilden.

2. Verfahren nach Anspruch 1, wobei das Fraktionieren des Stroms das Trennen von freien C₁₀- bis C₁₃-Fettsäuren als den ersten Teil von freien Fettsäuren und freien C₁₄₊-Fettsäuren als den zweiten Teil von freien Fettsäuren umfasst.

3. Verfahren nach Anspruch 1, wobei das Fraktionieren des Stroms das Trennen von freien C₁₀- bis C₁₃-Fettsäuren als den ersten Teil von freien Fettsäuren und Trennen von freien C₉₋-Fettsäuren und freien C₁₄₊-Fettsäuren als den zweiten Teil von freien Fettsäuren umfasst.

4. Verfahren nach Anspruch 1, wobei das Fraktionieren des Stroms das Trennen von freien C₁₀- bis C₁₃-Fettsäuren als den ersten Teil von freien Fettsäuren umfasst und wobei der erste Teil von freien Fettsäuren mindestens 97 Gew.-% freie C₁₀- bis C₁₃-Fettsäuren umfasst.

5. Verfahren nach Anspruch 4, wobei das Fraktionieren des Stroms das Trennen von freien C₁₀- bis C₁₃-Fettsäuren als den ersten Teil von freien Fettsäuren umfasst und wobei der erste Teil von freien Fettsäuren nicht mehr als 2 Gew.-% freie C₉₋-Fettsäuren umfasst.

6. Verfahren nach Anspruch 5, wobei das Fraktionieren des Stroms das Trennen von freien C₁₀- bis C₁₃-Fettsäuren als den ersten Teil von freien Fettsäuren umfasst und wobei der erste Teil von freien Fettsäuren nicht mehr als 1 Gew.-% freie C₁₄₊-Fettsäuren umfasst.

7. Verfahren nach Anspruch 1, ferner umfassend das Bereitstellen von Palmkernöl oder Kokosöl als natürliches ÖI.

8. Verfahren nach Anspruch 1, wobei das natürliche ÖI Fettsäuren mit internen Hydroxylgruppen umfasst und wobei die Desoxidation des natürlichen ÖIs eine Spaltung bewirkt und den ersten Teil von freien Fettsäuren und den zweiten Teil von freien Fettsäuren bereitstellt.

## Revendications

1. Procédé de co-production d'un produit alkylbenzène (11) et d'un produit oléochimique (12) à partir d'une huile naturelle (13), le procédé comprenant :
la lipolyse de l'huile naturelle pour former un flux d'acides gras libres (15) ;
le fractionnement du flux d'acides gras libres pour séparer une première partie (18) d'acides gras libres et une seconde partie (19) d'acides gras libres ;
le traitement de la première partie d'acides gras libres pour fournir le produit alkylbenzène ; et
le traitement de la seconde partie d'acides gras libres pour former le produit oléochimique ;
dans lequel le traitement de la première partie d'acides gras libres comprend :
la désoxygénation de la première partie d'acides gras libres pour produire des paraffines normales (28) ;
la déshydrogénation des paraffines normales pour fournir des mono-oléfines (52) ;
l'alkylation du benzène (54) avec les mono-oléfines dans des conditions d'alkylation pour fournir un effluent d'alkylation (60) comprenant des alkylbenzènes et du benzène ; et
l'isolement des alkylbenzènes pour fournir le produit alkylbenzène ; et
dans lequel le traitement de la seconde partie d'acides gras libres comprend la réalisation d'un processus d'estérification et d'un processus de sulfonation pour former un produit sulfonate d'ester méthylique.

2. Procédé selon la revendication 1 dans lequel le fractionnement du flux comprend la séparation des acides gras libres en C₁₀ à C₁₃ en tant que première partie d'acides gras libres et des acides gras libres en C₁₄₊ en tant que seconde partie d'acides gras libres.

3. Procédé selon la revendication 1 dans lequel le fractionnement du flux comprend la séparation des acides gras libres en C₁₀ à C₁₃ en tant que première partie d'acides gras libres, et la séparation des acides gras libres en C₉₋ et des acides gras libres en C₁₄₊ en tant que seconde partie d'acides gras libres.

4. Procédé selon la revendication 1 dans lequel le fractionnement du flux comprend la séparation des acides gras libres en C₁₀ à C₁₃ en tant que première partie d'acides gras libres et dans lequel la première partie d'acides gras libres comprend au moins 97 % en poids d'acides gras libres en C₁₀ à C₁₃.

5. Procédé selon la revendication 4 dans lequel le fractionnement du flux comprend la séparation des acides gras libres en C₁₀ à C₁₃ en tant que première partie d'acides gras libres et dans lequel la première partie d'acides gras libres ne comprend pas plus de 2 % en poids d'acides gras libres en C₉₋.

6. Procédé selon la revendication 5 dans lequel le fractionnement du flux comprend la séparation des acides gras libres en C₁₀ à C₁₃ en tant que première partie d'acides gras libres et dans lequel la première partie d'acides gras libres ne comprend pas plus de 1 % en poids d'acides gras libres en C₁₄₊.

7. Procédé selon la revendication 1 comprenant en outre la fourniture d'huile de palmiste ou d'huile de noix de coco en tant qu'huile naturelle.

8. Procédé selon la revendication 1 dans lequel l'huile naturelle comprend des acides gras avec des groupes hydroxyle internes, et dans lequel la désoxygénation de l'huile naturelle provoque le clivage et fournit la première partie d'acides gras libres et la seconde partie d'acides gras libres.
